# EUROPEAN PATENT APPLICATION

(11) **EP 3 903 668 A1**
(43) Date of publication of application: **03.11.2021**
(21) Application number: 20172496.0
(22) Date of filing: 30.04.2020
(51) Int. Cl.: A61B 5/00, G06K 9/00

(54) **A SYSTEM FOR PROVIDING GUIDANCE**

(71) Applicant: Koninklijke Philips N.V., 5656 AG Eindhoven (NL)
(72) Inventor: Bulut, Murtaza, 5656 AE Eindhoven (NL); Hilbig, Rainer, 5656 AE Eindhoven (NL); Shi, Jun, 5656 AE Eindhoven (NL); Palero, Jonathan Alambra, 5656 AE Eindhoven (NL); Zhang, Qiushi, 5656 AE Eindhoven (NL)
(74) Representative: Philips Intellectual Property & Standards

(57) **Abstract**

There is provided a computer-implemented for controlling a system, the method comprising: acquiring conditions under which data required for analyzing a first biological parameter is to be captured; determining, based on the conditions, requirements associated with an orientation of a body part, a position of a body part, a movement of a body part, and/or a level of a second biological parameter; acquiring, based on the requirements, a current orientation of the body part, a current position of the body part, a current movement of a body part of the subject, and/or a current level of the second biological parameter; determining a required adjustment of an orientation of the body part, a position of the body part, and/or a second biological parameter, based on the respective requirement and the information associated with the respective requirement; generating guidance for the subject based on the determined required adjustment.

## Description

### FIELD OF THE INVENTION

The present disclosure relates to a system configured to provide guidance to a subject, and a method for controlling thereof. The present disclosure also relates to a device comprising a reflective component and the system configured to provide guidance to a subj ect.

### BACKGROUND OF THE INVENTION

Image based analysis and monitoring of skin condition typically require a large amount of image data, preferably image data that is collected every day (or even more frequently). Some of the currently available techniques achieve this by collecting a large amount of image data whenever possible, e.g. when sensors detect the presence of a user, without performing any prior determination with regard to whether the data would be useful. This approach may be regarded as overly intrusive of users' privacy, as it usually results in collection of data that may not be necessary for the intended analysis.

An alternative currently available approach is to involve users directly and to request users' cooperation in the data collection, for example by asking users to adopt certain positions or orientations for the purpose of image capture, or asking users to change the lighting conditions in order for images to be captured. However, with this approach, since the data collection is dependent on the direct engagement with users, the users may be interrupted frequently and this may result in user dissatisfaction and annoyance. Furthermore, this may dissuade users from continuous or long-term use of the relevant system or application.

### SUMMARY OF THE INVENTION

Quantification and tracking of skin condition and skin treatments by means of image analyses (e.g. of the face of a subject) require a database that contains a large number of images that are acquired with similar angles, positions, and lighting conditions, as well as a large number of images that are acquired with different angles, positions, and lighting conditions.

As noted above, there are a number of disadvantages associated with the currently available solutions for data collection for the purpose of image-based analysis a monitoring of skin condition or other health conditions, for example some systems involve asking users to adopt certain positions and orientations before images are acquired, or acquiring images at all times, or acquiring images at pre-set intervals. The limitations of these approaches are that they are highly obtrusive, not privacy sensitive, or potentially missing important data. For personal care devices and applications, it is desired to collect representative data in a ubiquitous manner, i.e. in a manner that does not perceivably interrupt the main activity being carried out by the subject.

It would therefore be advantageous to provide an improved system configured to provide guidance to a subject, and a method for controlling thereof, which can allow data (e.g. image data) to be collected in an unobtrusive manner while also being privacy sensitive, by guiding subjects naturally to adopt a required position or orientation (e.g. with their body, head, torso, etc.), or perform a certain movement with their body part(s), before information is captured. Parts of the present disclosure relates to the use of a smart mirror for collecting mirror for collecting information relevant for skin condition or other health condition monitoring. Smart mirrors, or other types of smart, connected devices, are capable of adapting to users' changing needs and circumstances in terms of personal style and appearance and the process of aging. In addition to serving personal care needs, the systems and methods according to embodiments of the present disclosure can also discreetly measure and monitor aspects such as skin health, the condition of hair and teeth, the effects of lack of sleep, and pregnancy progress, movements of the subject (e.g. scratching of skin), emotions and mood variations, tiredness, frailty (especially for elderly subjects), diseases (e.g. Parkinson's disease), attention and responsiveness, alertness, etc.

To better address one or more of the concerns mentioned earlier, in a first aspect, a computer-implemented method for controlling a system configured to provide guidance to a subject is provided. The method comprises: acquiring one or more conditions under which data required for analyzing a first biological parameter of the subject is to be captured; determining, based on the one or more conditions, one or more requirements associated with at least one of: an orientation of a body part of the subject, a position of a body part of the subject, a movement of a body part of the subject, and a level of a second biological parameter of the subject; acquiring, based on the one or more requirements, at least one of: a current orientation of the body part of the subject, a current position of the body part of the subject, a current movement of a body part of the subject, and a current level of the second biological parameter of the subject; determining a required adjustment of at least one of: an orientation of the body part of the subject, a position of the body part of the subject, and a second biological parameter of the subject, based on the respective requirement and the acquired information associated with the respective requirement; generating guidance for the subject based on the determined required adjustment by performing at least one of: generating a directional sound effect to be outputted by a user interface of the system, wherein the directional sound effect is configured to induce the subject to achieve the required adjustment; determining at least one of a relative position and an attribute of a visual element that is to be outputted by a user interface of the system so as to induce the subject to achieve the required adjustment; and adjusting a setting of a sensing unit of the system to achieve the required adjustment.

In some embodiments, the relative position of a visual element may be represented by coordinates and may indicate a position of the visual element relative to one or more sensing units of the system. In these embodiments, determining at least a relative position of the visual element that is to be outputted by a user interface of the system comprises determining the coordinates of the visual element.

In some embodiments, determining an attribute of a visual element that is to be outputted by a user interface of the system may comprise determining at least one of: a viewing angle of the visual element, a viewing depth of the visual element, a viewing distance of the visual element, and a size of the visual element.

In some embodiments, determining at least one of a relative position and an attribute of a visual element that is to be outputted by a user interface may be further based on the first biological parameter.

In some embodiments where generating guidance for the subject based on the determined required adjustment comprises generating a directional sound effect, the method may further comprise determining an attribute of the directional sound effect.

In some embodiments, the method may further comprise, prior to acquiring one or more conditions under which the data required for analyzing a first biological parameter of the subject is to be captured, the steps of: acquiring initial data associated with at least one of the subject, a device used by the subject, and an application program used by the subject; and determining whether the acquired initial data is sufficient for performing an analysis of the first biological parameter with at least one of a level of accuracy higher than a predetermined threshold and a level of speed higher than a predetermined threshold. In these embodiments, acquiring one or more conditions under which the data required for analyzing a first biological parameter of subject is to be captured may be performed upon determining that the acquired initial data is not sufficient for performing an analysis of the first biological parameters with at least one of a level of accuracy higher than the predetermined threshold and a level of speed higher than the predetermined threshold.

In some embodiments, the acquired initial data may comprise at least one of: data associated with a preference of the subject, data associated with an environment of the subject, usage data of a device used by the subject, usage data of an application program used by the subject, a value of the second biological parameter, image data of a body part of the subject associated with the first biological parameter, and sound data generated by at least one of the subject, the environment of the subject, a device used by the subject, or an application program used by the subject.

In some embodiments, the method may further comprise acquiring at least one of: a current orientation of the body part of the subject, a current position of the body part of the subject, a current movement of a body part of the subject, and a current level of the second biological parameter of the subject comprises: acquiring at least one of: an image of the body part of the subject and a sound of the environment of the subject; detecting one or more physical features of the body part of the subject by performing analysis of the at least one of acquired image and a sound of the environment of the subject; and determining, based on the detected one or more physical features, the at least one of: a current orientation of the body part of the subject, a current position of the body part of the subject, a current movement of a body part of the subject, and a current level of the second biological parameter of the subject.

In some embodiments, the method may further comprise: acquiring feedback from the subject in response to the generated guidance, the feedback being associated with at least one of a movement of a body part of the subject and a third biological parameter of the subject; determining whether an update of the guidance is required based on the acquired feedback; and generating new guidance for the subject when it is determined that an update of the guidance is required.

In some embodiments, a biological parameter may be associated with one of the physiological state of the subject and the psychological state of the subject.

In some embodiments, the data required for analyzing a first biological parameter of a subj ect may comprise one or more images of the body part of the subject, and each of the one or more conditions under which data required for analyzing a first biological parameter of a subj ect is to be captured is associated with at least one of: a sharpness of the one or more images, a level of illumination of the body part of the subject, an angle at which the one or more images are captured, and an activity performed by the user during which the one or more images are captured.

In a second aspect, there is provided a computer program product comprising a computer readable medium, the computer readable medium having computer readable code embodied therein, the computer readable code being configured such that, on execution by a suitable computer or processor, the computer or processor is caused to perform the method as described herein.

In a third aspect, there is provided a system configured to provide guidance to a subject, the system comprising: a user interface configured to output one or more visual or audio elements; a sensing unit configured to capture data required for analyzing a first biological parameter of a subject; and a control unit configured to: acquire one or more conditions under which the data required for analyzing the first biological parameter is to be captured; determine, based on the one or more conditions, one or more requirements associated with at least one of: an orientation of a body part of the subject, a position of a body part of the subject, a movement of a body part of the subject, and a level of a second biological parameter of the subject; acquire, based on the one or more requirements, at least one of: a current orientation of the body part of the subject, a current position of the body part of the subject, a current movement of a body part of the subject, and a current level of the second biological parameter of the subject; determine a required adjustment of at least one of: an orientation of the body part of the subject, a position of the body part of the subject, and a second biological parameter of the subject, based on the respective requirement and the acquired information associated with the respective requirement; generate guidance for the subject based on the determined required adjustment by performing at least one of: generating a directional sound effect to be outputted by the user interface, wherein the directional sound effect is configured to induce the subject to achieve the required adjustment; determining at least one of a relative position and an attribute of a visual element that is to be outputted by the user interface so as to induce the subject to achieve the required adjustment; and adjusting a setting of the sensing unit to achieve the required adjustment.

In some embodiments, the control unit may be further configured to, prior to acquiring one or more conditions under which the required data is to be captured, acquire initial data associated with at least one of the subject, a device used by the subject, and an application program used by the subject, and determine whether the acquired initial data is sufficient for performing an analysis of the first biological parameter with at least one of: a level of accuracy higher than a predetermined threshold and a level of speed higher than a predetermined threshold. Furthermore, the control unit may be further configured to acquire one or more conditions under which the data required for analyzing a first biological parameter of subject is to be captured when it is determined that the acquired initial data is not sufficient for performing an analysis of the first biological parameters with at least one of: a level of accuracy higher than the predetermined threshold and a level of speed higher than a predetermined threshold.

In a fourth aspect, there is provided a device comprising a reflective component configured to reflect incident light, and the system as described herein.

According to the aspects and embodiments described above, the limitations of existing techniques are addressed. In particular, the above-described aspects and embodiments enable guidance to be provided to subjects to induce the subjects to move or behave in a way such that desired conditions can be achieved for data collection. The embodiments described above offer a number of different ways that such guidance can be generated and provided to "nudge" the subjects. In this way, desired data can be collected in a ubiquitous and unobtrusive manner.

There is thus provided an improved system configured to provide guidance to a subject, and a method for controlling thereof. These and other aspects of the disclosure will be apparent from and elucidated with reference to the embodiment(s) described hereinafter.

### BRIEF DESCRIPTION OF THE DRAWINGS

For a better understanding of the embodiments, and to show more clearly how they may be carried into effect, reference will now be made, by way of example only, to the accompanying drawings, in which:
Fig. 1 is a block diagram of a system which can be configured to provide guidance to a subject, according to an embodiments;
Fig. 2 illustrates a computer-implemented method for controlling a system configured to provide guidance to a subject, according to an embodiment; and
Fig. 3 illustrates an implementation of determining different relative positions of a visual element outputted by a user interface, according to an embodiment.

### DETAILED DESCRIPTION OF THE EMBODIMENTS

As noted above, there is provided an improved system and a method for controlling the same, which address the existing problems.

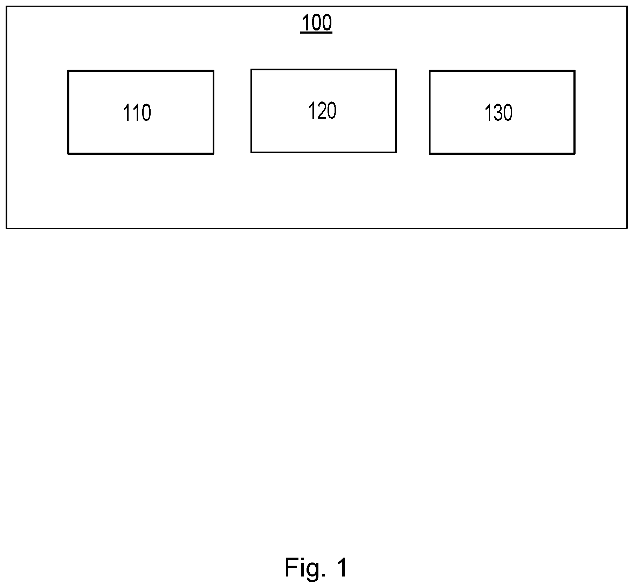

Fig. 1 shows a block diagram of a system 100 according to an embodiment, which can be configured to provide guidance to a subject. The guidance can be implicit or explicit resulting in the subject making conscious or unconscious changes or adaptations in terms of posture and/or positioning of their body or body part(s). The system can induce one-way interaction as well as two-way interaction with the subject, as will be explained in more detail in the following.

Although the operation of the system 100 is described below in the context of a single subject, it will be appreciated that the system 100 is capable of providing guidance to a plurality of subjects. Also, although embodiments described herein may be described with reference to the collection of image data, it will be appreciated that the present embodiments are applicable with respect to other types of data, e.g. speech of the subject as well as other sounds. In addition, although the operation of the system 100 is described below in the context of personal health domain, especially skin care and dental care, it will be appreciated that the system 100 may be used in applications that requires acquisition of image data or other types of data).

As illustrated in Fig. 1, the system 100 comprises a user interface 110, a sensing unit 120, and a control unit 130.

The user interface 110 is configured to output one or more visual or audio elements. In some embodiments, the user interface 110 may comprise a wearable unit configured to output at least one of: one or more visual elements and one or more audio elements. The wearable unit may be configured to output one or more visual elements in at least one of a 3D format, a holographic format, a virtual reality (VR) format, and an augmented reality (AR) format. In some embodiments, the user interface 110 may adopt liquid lens technology for outputting the one or more visual elements.

Moreover, the user interface 110 may be any user interface that enables the rendering (or output or display) of information to a user of the system 100 in the audio or visual format. Additionally, the user interface 110 may be a user interface that enables a user (e.g. the subject) of the system 100 to provide a user input, interact with and/or control the system 100. For example, the user interface 110 may comprise one or more switches, one or more buttons, a keypad, a keyboard, a touch screen or an application (for example, on a tablet or smartphone), a display screen, a graphical user interface (GUI) or other visual rendering component, one or more speakers, one or more microphones or any other audio component, one or more lights, a component for providing tactile feedback (e.g. a vibration function), or any other user interface, or combination of user interfaces.

The sensing unit 120 is configured to capture data required for analyzing a first biological parameter of a subject. The sensing unit 120 may comprise at least one of a camera and a wearable sensor (e.g. for skin conductance, heart rate, temperature, sound, movement, etc.) in some embodiments. A biological parameter may be associated with one of the physiological state of the subject and the psychological state of the subject.

The control unit 130 may control the operation of the system 100 and can implement the method described herein. The control unit 130 can comprise one or more processors, processing units, multi-core processor or modules that are configured or programmed to control the system 100 in the manner described herein. In particular implementations, the control unit 130 can comprise a plurality of software and/or hardware modules that are each configured to perform, or are for performing, individual or multiple steps of the method described herein.

Briefly, the control unit 130 is configured to acquire one or more conditions under which the data required for analyzing the first biological parameter is to be captured, and determine, based on the one or more conditions, one or more requirements associated with at least one of: an orientation of a body part of the subject, a position of a body part of the subject, a movement of a body part of the subject, and a level of a second biological parameter of the subject. The control unit 130 is also configured to acquire, based on the one or more requirements, at least one of: a current orientation of the body part of the subject, a current position of the body part of the subject, a current movement of a body part of the subject, and a current level of the second biological parameter of the subject. The control unit 130 is configured to then determine a required adjustment of at least one of: an orientation of the body part of the subject, a position of the body part of the subject, and a second biological parameter of the subject, based on the respective requirement and the acquired information associated with the respective requirement.

The control unit 130 is further configured to generate guidance for the subject based on the determined required adjustment by performing at least one of: generating a directional sound effect to be outputted by the user interface, wherein the directional sound effect is configured to induce the subject to achieve the required adjustment, determining at least one of a relative position and an attribute of a visual element that is to be outputted by the user interface so as to induce the subject to achieve the required adjustment, and adjusting a setting of the sensing unit to achieve the required adjustment. In some embodiments, the visual element may be provided in the form of an active window displayed via the user interface 110.

In some embodiments, the control unit 130 may be further configured to, prior to acquiring one or more conditions under which the required data is to be captured, acquire initial data associated with at least one of the subject, a device used by the subject, and an application program used by the subject, and determine whether the acquired initial data is sufficient for performing an analysis of the first biological parameter with at least one of: a level of accuracy higher than a predetermined threshold and a level of speed higher than a predetermined threshold. The relevant predetermined threshold(s) may be static thresholds or dynamic thresholds. In these embodiments, the control unit 130 may be configured to acquiring one or more conditions under which the data required for analyzing a first biological parameter of subject is to be captured when it is determined that the acquired initial data is not sufficient for performing an analysis of the first biological parameters with at least one of: a level of accuracy higher than the predetermined threshold and a level of speed higher than a predetermined threshold.

In some embodiments, the control unit 130 may be configured to perform the analysis of the first biological parameter based on the captured data under the acquired one or more conditions (with or without initial data).

Moreover, the control unit 130 may be configured to acquire at least one of: a current orientation of the body part of the subject, a current position of the body part of the subject, a current movement of a body part of the subject, and a current level of the second biological parameter of the subject by performing at least one of: gesture recognition, facial expression recognition, speech recognition, emotion recognition (e.g. from speech or image) of data captured by the sensing unit 120.

In some embodiments, the user interface 110 may be part of another device separate from the sensing unit 120 and the control unit 130. The user interface 110 may be for use in providing a user of the apparatus 100 with information resulting from the method described herein. In addition, the user interface 110 may be configured to receive a user input. For example, the user interface 110 may allow a user of the system 100 to manually enter instructions, data, or information. In these embodiments, the control unit 130 may be configured to acquire the user input from the user interface 110.

Although not shown in the drawing, the system 100 may comprise a memory. Alternatively or in addition, one or more memories may be external to (i.e. separate to or remote from) the system 100. For example, one or more memories may be part of another device. A memory can be configured to store program code that can be executed by the control unit 130 to perform the method described herein. A memory can be used to store information, data, signals and measurements acquired or made by the control unit 130 of the system 100. For example, a memory may be used to store (for example, in a local file) the acquired one or more conditions under which the data required for analyzing the first biological parameter is to be captured. The control unit 130 may be configured to control a memory to store the acquired one or more conditions under which the data required for analyzing the first biological parameter is to be captured.

Although not shown in the drawing, the system 100 may comprise a communications interface (or circuitry) for enabling the system 100 to communicate with any interfaces, memories and/or devices that are internal or external to the system 100. The communications interface may communicate with any interfaces, memories and/or devices wirelessly or via a wired connection. For example, the communications interface may communicate with the user interface 110 wirelessly or via a wired connection. Similarly, the communications interface may communicate with the one or more memories wirelessly or via a wired connection.

It will be appreciated that Fig. 1 only shows the components required to illustrate an aspect of the system 100 and, in a practical implementation, the system 100 may comprise alternative or additional components to those shown.

Moreover, in some embodiments, there may be provided a device comprising a reflective component configured to reflect incident light, and the system 100 as described herein. For example, the device may be a smart mirror. In this example, the user interface 110 may be a touch screen incorporated at the reflective (mirror) component and the sensor unit 120 may comprise a camera incorporated at the reflective component of the smart mirror. The device may be part of a network (e.g. implemented in the subject's bathroom) comprising additional components that can complement the device and the system 100 in terms of health diagnostics as well as improved primary performance of personal care products, styling advice, and other personal health or grooming related services. In addition, in some embodiments, there may be provide a personal care device or a personal health device comprising the system 100 as described herein.

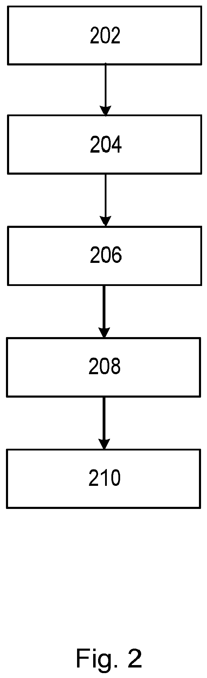

Fig. 2 illustrates a computer-implemented method for controlling a system configured to provide guidance to a subject, according to an embodiment. The illustrated method can generally be performed by or under the control of the control unit 130 of the system 100. The illustrated method will be described below with reference to the system 100 of Fig. 1 and its components.

With reference to Fig. 2, at block 202, one or more conditions under which data required for analyzing a first biological parameter of the subject is to be captured are acquired. As explained with reference to Fig. 1 above, a biological parameter may be associated with one of the physiological state of the subj ect and the psychological state of the subject. The first biological parameter may be, for example in the context of skin analysis, a level of oiliness of the skin of the subject, or a level of elasticity of the skin of the subject, or a parameter relating to a condition (e.g. acne, eczema) on the face of the subject, such as a level of redness of an eczema region. As another example, the first biological parameter may be, in the context of dental analysis, the extent of plague on the teeth of the subject. The data required for analysis may comprise measured values of other biological parameter(s), for example the current blood sugar level of the subject, one or more images, one or more videos, etc.

In some embodiments, the data required for analyzing a first biological parameter of a subject may comprise one or more images of the body part (e.g. face, hands, or teeth) of the subject. In these embodiments, each of the one or more conditions under which data required for analyzing a first biological parameter of a subject is to be captured may be associated with at least one of: a sharpness of the one or more images, a level of illumination of the body part of the subject, an angle at which the one or more images are captured, and an activity performed by the user during which the one or more images are captured. This is because typically a large number of temporally distributed high quality representative images are required for the analysis of a relevant biological parameter, where the quality may be indicative of the representativeness with respect to the relevant analysis, in addition to sharpness, content, color, etc. For example, high quality images of the left cheek of the subject are required for analyzing an eczema spot on the left cheek of the subject such that region boundaries of the eczema spot can be identified. In addition, in some cases past images with similar lighting conditions and/or angles may be required such that a level of redness and/or a level of roughness of the eczema region can be quantified. Furthermore, images and/or videos of the subject interacting with the eczema region, e.g. scratching or touching as well as the extent or strength of the scratching or touching (whether any scratch marks were made, etc.), may be acquired for analyzing a level of irritation, or pain, or discomfort caused by the eczema condition.

In some embodiments, prior to acquiring one or more conditions under which the data required for analyzing a first biological parameter of the subj ect is to be captured at block 202, the method may comprise performing the steps of: acquiring initial data associated with at least one of the subject, a device used by the subject, and an application program used by the subject, and determining whether the acquired initial data is sufficient for performing an analysis of the first biological parameter with at least one of: a level of accuracy higher than a predetermined threshold and a level of speed higher than a predetermined threshold. The relevant predetermined threshold(s) may be static or dynamic.

In some cases, it may be determined that the acquired initial data is not sufficient for performing an analysis of the first biological parameter (e.g. a level of oiliness of the skin of the subject) with at least one of: a level of accuracy higher than a predetermined threshold and a level of speed higher than a predetermined threshold. In such cases, the method may comprise acquiring further initial data until it can be determined that the cumulative initial data acquired is sufficient for performing an analysis of the first biological parameter with at least one of: a level of accuracy higher than a predetermined threshold and a level of speed higher than a predetermined threshold. The analysis of the first biological parameter may be an immediate (e.g. real-time, or close to real-time) analysis or a scheduled analysis. For example, the subject may be concerned with treatment for a skin condition and the treatment may last weeks - in this example the control unit 130 may predict that a certain type of information would be required for the scheduled analysis in the future. This way, data is not only acquired for a current analysis of the first biological parameter, but also acquired predictively for a scheduled analysis to be carried out (for example to track a progress of a treatment of a skin condition).

In these embodiments, acquiring one or more conditions under which the data required for analyzing a first biological parameter of subject is to be captured at block 202 may be performed upon determining that the acquired initial data is not sufficient for performing an analysis of the first biological parameters with at least one of: a level of accuracy higher than the predetermined threshold and a level of speed higher than the predetermined threshold.

Moreover, in these embodiments, the acquired initial data may comprise at least one of: data associated with a preference of the subject, data associated with an environment of the subject, usage data of a device used by the subject, usage data of an application program used by the subject, a value of the second biological parameter, image data of a body part of the subject associated with the first biological parameter, and sound data (e.g. speech) generated by at least one of: the subject, the environment of the subject, a device used by the subject, or an application program used by the subject.

For example, data associated with a preference of the subject may include information relating to time and/or date preferred by the subject with respect to a device associated with or comprising the system 100, for example a smart mirror. Data associated with a preference of the subject may additionally or alternatively include physiological parameter(s) of the subject, e.g. heart rate, whether the subject prefers to make movements during certain activities, whether the subject prefers audio or visual stimulation during certain activities, a type of content preferred by the subject, etc. Data associated with an environment of the subject may, for example, include location data and/or local weather information. The device used by the subject may be a personal care device (e.g. an electric toothbrush, a skincare device, an electric face brush), or a personal health device (e.g. a weighing scale), or a device associated with an aspect of the subject's health (e.g. a cooker which can provide nutrition information of the food items consumed by the subject). The application program used by the subject may be, for example, an application program associated with a personal care device, or a personal health device, or a device associated with an aspect of the subject's health. Alternatively or in addition, the application program used by the subject may be a smartphone application for monitoring health (e.g. oral health, sleep analysis) of the subject.

Acquiring the initial data may comprise receiving the initial data through an input by the subject via the user interface 110 of the system 100. In some embodiments, the initial data may be learned automatically via interaction with the subject by the system 100.

Returning to Fig. 2, at block 204, one or more requirements associated with at least one of: an orientation of a body part of the subject, a position of a body part of the subject, a movement of a body part of the subject, and a level of a second biological parameter of the subject are determined. This determination is based on the one or more conditions acquired at block 202. In some embodiments, the determination of the one or more requirements at block 204 may be further based on a current activity being performed by the subj ect.

Returning to Fig. 2, at block 206, at least one of: a current orientation of the body part of the subject, a current position of the body part of the subject, a current movement of a body part of the subject, and a current level of the second biological parameter of the subject is acquired. This acquisition is based on the one or more requirements determined at block 204.

In some embodiments, at block 206, acquiring at least one of: a current orientation of the body part of the subject, a current movement of a body part of the subject, a current position of the body part of the subject, and a current level of the second biological parameter of the subject may comprise: acquiring at least one of: an image of the body part of the subject and a sound of the environment of the subject, detecting one or more physical features of the body part of the subj ect by performing analysis of at least one of the acquired image and the acquired sound, and determining, based on the detected one or more physical features, the at least one of: a current orientation of the body part of the subject, a current position of the body part of the subject, a current movement of a body part of the subject, and a current level of the second biological parameter of the subject.

Returning to Fig. 2, at block 208, a required adjustment of at least one of: an orientation of the body part of the subject, a position of the body part of the subject, and a second biological parameter of the subject is determined. This determination is based on the respective requirement determined at block 204 and the information associated with the respective requirement acquired at block 206.

Returning to Fig. 2, at block 210, guidance for the subject is generated based on the required adjustment determined at block 208. The generation of guidance is achieved by performing at least one of:
- generating a directional sound effect to be outputted by a user interface of the system, wherein the directional sound effect is configured to induce the subject to achieve the required adjustment;
- determining at least one of a relative position and an attribute (e.g. viewing angle, depth, etc.) of a visual element (e.g. an active window) that is to be outputted by a user interface of the system so as to induce the subject to achieve the required adjustment; and
- adjusting a setting of a sensing unit of the system to achieve the required adj ustment.

As an example, guidance for the subject can be generated by generating a directional sound effect to induce the subject to move their head to achieve the required adjustment. For example, a directional sound effect can be provided using beam forming, which can induce the subject to turn their head in the direction towards the sound and enable image capture of one side of the head of the subject. In some embodiments where the method includes generating a direction sound effect, the method may further comprise determining an attribute of the directional sound effect. The attribute of the sound effect may be associated with at least one of: pitch, loudness, tempo, and silence or pause period(s).

As an example, guidance for the subject can be generated by determining the relative position of a visual element, e.g. a small red dot, to be outputted by a user interface of the system which induces the subject to look towards a specific direction and enable image capture of a side profile of the subject. Alternatively, the relative position of a visual element may be determined to induce the subject to perform a swiping action such that the hand and arms of the subject are in place at the appropriate angles and appropriate lighting conditions for image capture.

In some embodiments, analysis techniques (e.g. image analysis techniques) can be employed in determining at least one of a relative position and an attribute of a visual element. This may be performed under the assumption that the position and the orientation of the sensing unit is fixed. Image recognition can be used to locate pixel(s) corresponding to a body feature (e.g. head, shoulder, eyes, nose, ears, etc.) of the subject in a captured image of the subject. A relative position of the visual element can be determined such that coordinates representing the relative position align with coordinates of the pixel(s) corresponding to the body feature. Moreover, in some cases, by determining the distance between pixel(s) corresponding to a left ear and pixel(s) corresponding to a right ear of the subject, distance of the subject from the user interface (and/or the sensing unit) may be determined for the purpose of setting a baseline. In this way, if the subject is not positioned according to one or more of the requirements determined at block 204, the visual element may be blurred (the blurring effect being a determined attribute).

As another example, guidance for the subject can be generated by adjusting setting of the sensing unit (e.g. a camera) such as zooming in or zooming out and/or adjusting the position and/or orientation, alone or in addition to determining a relative position of a visual element that is to be outputted by the user interface (this may depend on the limitations of the adjustment of the settings of the sensing unit).

In some embodiments, determining a relative position of a visual element that is to be outputted by a user interface of the system may comprise selecting a set of relative positions from a plurality of candidate sets of relative positions, and determining the relative position from the selected candidate set. In these embodiments, each of the plurality of candidate sets may be associated with a biological parameter, a health condition, and/or a treatment. For example, candidate set 1 of relative positions may be associated with analysis of skin color (as a biological parameter), and candidate set 2 may be associated with analysis, monitoring, and treatment of wrinkles. The candidate sets may be determined based on previous data associated with one or more subjects, and/or based on population data of subjects with similar body and head dimensions.

Similarly, in some embodiments, determining an attribute of a visual element that is to be outputted by a user interface of the system may comprise selecting a set of attributes from a plurality of candidate sets of attributes, and determining the attribute from the selected candidate set. Also, similarly, in some embodiments, adjusting a setting of the sensing unit may comprise selecting a set of sensing unit settings from a plurality of candidate sets of sensing unit settings, and determining the setting to be adjusted from the selected candidate set.

In some embodiments, the content associated with the visual element may include non-instructional prompt(s) to guide the subject to perform the required adjustment. For example, a video (as at least a part of the visual element) of people yawning may be shown so as to induce the subject to yawn, which may be useful in assessing skin elasticity of the face of the subject. The same principle can be applied for laughter (e.g. video of people laughing, or sound clips of people laughing) to induce the subject to smile or laugh, which may be useful to relax the subj ect resulting in desired data being captured for the analysis of the first biological parameter.

In some embodiments where the generation of guidance at block 210 is achieved by at least determining at least one of a relative position and an attribute of a visual element that is to be outputted by a user interface of the system, the visual element may be provided in at least one of: a 3D format, a holographic format, a virtual reality format, and an augmented reality format. In particular, in embodiments where the visual element is provided in a 3D format, attribute(s) of the visual element may be determined to motivate the subject to view from different angles and hence allowing images of their head to be captured from different angles by the sensing unit. In embodiments where the visual element is provided in a holographic format, the visual element may be provided such that content is only viewable from a certain angle so as to induce the subject to assume a certain position and/or orientation with their body part(s) or to perform a certain movement with their body part(s). Furthermore, in some embodiments where the user interface 110 of the system 100 comprises a wearable unit, the relative position and an attribute of a visual element outputted via such wearable unit can be determined accordingly to induce the subject to adopt a certain position and/or orientation or to perform a certain movement of a body part. Also, in some embodiments where the user interface 110 of the system 100 uses liquid lens technology in its output, an attribute relating to liquid lens display may be determined to induce the subject to achieve the required adjustment.

In some embodiment, the generation of guidance at block 210 may be further based on the first biological parameter. For example, if the first biological parameter is the level of redness of a region of the skin on the left cheek of the subject, then the relative position of the visual element may be determined in a way so as to induce the subj ect to turn their head such that their left cheek is in view of the sensing unit (e.g. a camera). Alternatively or additionally, a directional sound effect may be generated to induce the subj ect to turn their head.

Furthermore, the relative position and/or attribute(s) of the visual element, or the settings of the sensing unit, may be adjusted periodically (e.g. every day) to satisfy changing requirements with respect to at least one of: an orientation of the body part of the subject, a position of the body part of the subject, and a second biological parameter of the subject. These changing requirements may vary due to changing conditions under which data required for analyzing a first biological parameter of the subject is to be captured. For example, the relative positions of a visual element may be adjusted periodically to induce the subject to move their head towards different directions, such that images of the face of the subject to be captured by the sensing unit at different angles. As another example, the viewing angle of a visual element may be narrowed to induce the subject to position themselves at a certain angle with respect to the user interface (and accordingly the sensing unit), and/or the viewing distance of a visual element may be limited to induce the subject to positioned themselves at a certain range of distances with respect to the user interface (and accordingly the sensing unit). The adjustment of the relative position and/or attribute(s) of the visual element may be performed such that the adjustment may not be perceptible to the subject, e.g. changing the relative position of the visual element by a small vertical or horizontal shift. In the case where the visual element that is provided in a 3D format, the 3D attribute of the visual element may be adjusted in terms of variations in the depth perception.

In some cases, a small shift in terms of relative position of the visual element may not necessarily induce the subject to achieve the determined required adjustment with respect to an orientation of the body part of the subject, a position of the body part of the subject, and/or a second biological parameter of the subject. For example, the small shifts may only cause eye saccades or quick eye gaze by the subject without head movement. In fact, any shift of gaze larger than about 20° would be accompanies by head movement. For this reason, adjustments of the relative position of the visual element may not necessarily be a subtle "nudge", as large shifts may be required to induce the subject to achieve the required adjustment in terms of orientation of the body part and/or position of the body part and/or second biological parameter. Alternatively, if larger adjustments of the relative position of the visual element are not possible, for example due to physical constraints of the user interface or the environment of the subject, or constraints related to the health of the subject, or due to preferences of the subject, the attribute(s) of the visual element may be adjusted to induce the subject to achieve the required adjustment in terms of orientation of the body part and/or position of the body part and/or second biological parameter. In some embodiments, adjustments of both the relative position of the visual element and one or more attributes (e.g. viewing angle) of the visual element are possible.

In some embodiments, determining the relative position of the visual element may be based on information acquired from a device that is used by the subject, e.g. a smart tile or a weighing scale. For example, data associated with the left-right or front-back balance of the subject may be used for determining the relative position of the visual element to be outputted.

In embodiments where the generation of guidance at block 210 is achieved by at least determining a relative position of a visual element that is to be outputted by a user interface 110 of the system 100 so as to induce the subject to achieve the required adjustment, the relative position may be represented by coordinates (e.g. coordinates on a Cartesian plane). Moreover, the relative position may indicate a position of the visual element relative to one or more sensing units 120 of the system 100. Furthermore, in these embodiments, the determination of at least one of a relative position of a visual element that is to be outputted by a user interface 110 of the system 100 may comprise determining the coordinates of the visual element.

The determination of a relative position of a visual element that is to be outputted by a user interface 110 of the system 100 may be based on previous relative position(s) of the visual element. For example, the visual element may be an active window outputted via the user interface 110. In this example, the relative position (which may be presented by coordinates) may be stored in a memory and later retrieved when the same active window or a similar active window is to be outputted. The determination of the relative position of the (new) active window may be the same as the previous relative positon, or it may be based on the previous relative position with further adjustment. The further adjustment may be based on changing requirements with respect to the orientation of a respective body part, the position of a respective body part, and a level of the second biological parameter.

In addition, in embodiments where the generation of guidance at block 210 is achieved by at least determining at least one of a relative position of a visual element that is to be outputted by a user interface of the system, determining the relative position may comprise determining a default relative position of the visual element for the subject. The default relative position may be represented by coordinates, and the determination of a default relative position of the visual element for the subject may be based on factors such as body posture of the subject, one or more facial features of the subject, the height of the subject, etc. Such information may be acquired together with the at least one of: a current orientation of the body part of the subject, a current movement of a body part of the subject, a current position of the body part of the subject, and a current level of the second biological parameter of the subject at block 206. It will be appreciated that such information may be acquired at any point prior to determination of the default relative position of the visual element for the subject.

In embodiments where the generation of guidance at block 210 is achieved by at least determining at least an attribute of a visual element that is to be outputted by a user interface of the system so as to induce the subject to achieve the required adjustment, determining the attribute may comprise determining at least one of: a viewing angle of the visual element, a size of the visual element, a viewing depth of the visual element, and a viewing distance of the visual element.

Although not shown in the drawing, in some embodiments the method may further comprise the steps of: acquiring feedback from the subject in response to the generated guidance, the feedback being associated with at least one of a movement of a body part of the subject and a third biological parameter of the subject, determining whether an update of the guidance is required based on the acquired feedback, and generating new guidance for the subject when it is determined that an update of the guidance is required. The feedback may be acquired by the sensing unit 120 of the system 100. Furthermore, in these embodiments, the third biological parameter may be associated with one of the physiological state (e.g. heart rate) of the subject and the psychological state (e.g. an emotion as indicated by the facial expressions) of the subject. The movement of a body part of the subject may be for example a movement of the head of the subject in response to a generated visual element.

The purpose of acquiring such feedback is to monitor the response of the subject to the generated guidance to determine if the subject is able to perform their main activity without any distractions that may be caused by the generated guidance.

In an example, an attribute (e.g. viewing depth) of a visual element (e.g. an active window) may be determined and subsequently effected in the output of the visual element. In this example, the movement of the subject may be monitored as feedback to detect whether the subject has difficulties in adapting to the determined attribute of the visual element, e.g. whether the subject is moving backwards and forwards which prevents them from maintaining a correct distance from the user interface and from viewing the visual element clearly and sharply. In this case, instead of forcing the subject to move and assume a potentially uncomfortable position, at least one of the relative position and an attribute of the visual element can be further adjusted. In more detail, new relative position(s) of the visual element may be determined such that, instead of nudging the subject to move closer to the user interface, the position(s) would nudge the subject turn their head slightly left and then subsequently slightly right. In this way, image(s) that are captured when the subject has turned their head left can be used together with image(s) that are captured when the subject has turned their head right for the purpose of analyzing the first biological parameter. These images may be considered equivalent, in terms of usefulness of the intended analysis, to images that would be captured if the subject was positioned closer to the user interface. In other words, in this example a different data acquisition method is used for the purpose of analyzing the first biological parameter. The differences between the two acquisition methods are that the latter method would require more images to be captured as well as more time for the images to be captured. Nevertheless, the latter method provides the advantage that the subject is more comfortable while being able to view and potentially interact with the visual element more conveniently.

In another example, feedback relating to the physiological state of the subject may be acquired, such as a parameter related to fatigue (e.g. heart rate, respiration rate, etc.) can be determined via the sensing unit 120 of the system 100. The sensing unit 120 may comprise a camera which can track a movement of the chest of the subject to determine a respiration rate, for example. Alternatively or in addition, the sensing unit 120 may comprise a wearable sensor which allows heart rate of the subject to be detected. The method in these embodiment may further comprise determining a level of tiredness based on at least one of the detected heart rate and the detected respiration rate, and determining that an update of the guidance is required based on the level of tiredness. The new guidance may be generated by way of adjusting at least one of a relative position and an attribute of a visual element.

In yet another example, feedback relating to the psychological state (e.g. emotions) of the subject may be acquired. For example, feedback can be acquired via monitoring of facial expressions of the subject using a camera (which may be part of the sensing unit 120 of the system 100), or monitoring signals from a wearable sensor (e.g. skin conductance, heart rate variability, etc.). For example, a valence value of the emotion of the subject may be determined based on at least one of facial expressions and signals from a wearable sensor, and if the valence value is determined to be under a predetermined threshold it may be determined that an update of the guidance is required.

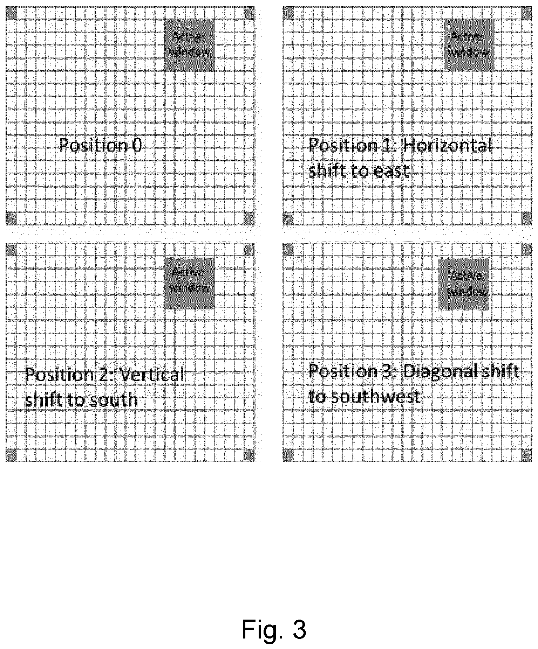

Fig. 3 illustrates an implementation of determining different relative positions of a visual element outputted by a user interface, according to an embodiment. In this embodiment, the visual element is an active window displayed at a screen implemented at a smart mirror device for the purpose of providing information (e.g. current weather, personal care data, skincare instructions, etc.) to a subject, the screen being part of the user interface of the system. The active window may include content consisting of text and/or graphics (images) among other data. The content of the active window by only be (best) viewable when the face (especially eyes and nose) and/or the body (especially shoulder blades) are in a certain position and/or orientation with respect to the relative position of the active window displayed on the screen. For example, the active window may be displayed in a way such that the information presented at the active window is only (best) readable when the eyes of the subject are aligned with the relative position of the active window displayed on the screen. Therefore, by adjusting or changing the different relative positions of the active window on the screen, the subject can be induced to adopt the appropriate or ideal positions and/or orientations with their body parts or perform a movement with respect to presumably fixed lighting conditions and fixed position of the sensing unit such that data can be collected seamlessly.

Four of the different relative positions that can be adopted by the active window in the present embodiment are illustrated in Fig. 3. To facilitate explanation of the relative positions, the screen is represented by a grid where the four corners are shaded to clearly indicate a relative position of the active window with reference to the corners of the screen. In some embodiments, each of the corners may be provided with a sensing unit, for example a camera to capture image data of a subject standing in front of the smart mirror device. It will be appreciated that in practical implementations the grid may not be outputted via the screen.

As shown in Fig. 3, the active window may initially adopt a default relative position ("Position 0") which is positioned near a top right corner of the screen. As described above with reference to Fig. 2, this default relative position may be based on factors such as body posture of the subject, one or more facial features of the subject, the height of the subject, etc.

Moreover, as explained above with reference to Fig. 2, the relative position of the active window may be adapted or adjusted depending on the requirements associated with at least one of: a current orientation of the body part of the subject, a current position of the body part of the subject, a current movement of a body part of the subject, and a current level of the second biological parameter of the subject. These requirements may vary depending on changing conditions under which data required for analyzing a first biological parameter of the subject is to be captured. For example, the relative position of the active window may be shifted to "east" (represented by a direction to the right), i.e. from the default relative position ("Position 0") to a new position ("Position 1") in order to induce the subject to move their head towards right. Similarly, the relative position of the active window may be shifted "south" (represented by a downward direction) or "southwest" (represented by a downward direction together with a direction to the left), i.e. from the default relative position ("Position 0") to a new position ("Position 2" or "Position 3") in order to induce the subject to tilt their head downwards (and towards the left). Since the shifts in relative position as demonstrated in Fig. 3 as explained above are relatively small, the change may not be perceptible by the subj ect.

There is thus provided an improved system configured to provide guidance to a subject, and a method for controlling thereof, which overcome the existing problems. The guidance generated using the systems and methods as described herein allow data collection to be performed in an unobtrusive and ubiquitous manner, since subject(s) would be subtly "nudged" to move or behave in a certain way that would be appropriate for data collection instead of being asked directly to perform certain actions. The "nudges" (guidance) may be determined based on a current activity being performed by the subject, and require voluntary cooperation from the subject in the case that ideal conditions for data collection are desired. Since the adaptations required by the subjects are not physically or mentally demanding, it is expected that the guidance generated would not cause any interruption or annoyance to the subjects and therefore cooperation can be facilitated. It is noted that in the context of the present disclosure, "unobtrusive and ubiquitous manner" may not necessarily exclude perceptible guidance. In some instances, the generated guidance may be perceptible by subjects.

There is also provided a computer program product comprising a computer readable medium, the computer readable medium having computer readable code embodied therein, the computer readable code being configured such that, on execution by a suitable computer or processor, the computer or processor is caused to perform the method or methods described herein. Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method for controlling a system configured to provide guidance to a subject, the method comprising:
acquiring (202) one or more conditions under which data required for analyzing a first biological parameter of the subject is to be captured;
determining (204), based on the one or more conditions, one or more requirements associated with at least one of: an orientation of a body part of the subject, a position of a body part of the subject, a movement of a body part of the subject, and a level of a second biological parameter of the subject;
acquiring (206), based on the one or more requirements, at least one of: a current orientation of the body part of the subject, a current movement of a body part of the subject, a current position of the body part of the subject, and a current level of the second biological parameter of the subject;
determining (208) a required adjustment of at least one of: an orientation of the body part of the subject, a position of the body part of the subject, and a second biological parameter of the subject, based on the respective requirement and the acquired information associated with the respective requirement;
generating (210) guidance for the subject based on the determined required adjustment by performing at least one of:
generating a directional sound effect to be outputted by a user interface of the system, wherein the directional sound effect is configured to induce the subject to achieve the required adjustment;
determining at least one of a relative position and an attribute of a visual element that is to be outputted by a user interface of the system so as to induce the subject to achieve the required adjustment; and
adjusting a setting of a sensing unit of the system to achieve the required adjustment.

2. The method according to claim 1, wherein the relative position of a visual element is represented by coordinates and indicates a position of the visual element relative to one or more sensing units of the system, and determining at least a relative position of the visual element that is to be outputted by a user interface of the system comprises determining the coordinates of the visual element.

3. The method according to claim 1 or claim 2, wherein determining an attribute of a visual element that is to be outputted by a user interface of the system comprises determining at least one of: a viewing angle of the visual element, a viewing depth of the visual element, a viewing distance of the visual element, and a size of the visual element.

4. The method according to any one of the preceding claims, wherein determining at least one of a relative position and an attribute of a visual element that is to be outputted by a user interface is further based on the first biological parameter.

5. The method according to any one of the preceding claims, wherein generating (210) guidance for the subject based on the determined required adjustment comprises generating a directional sound effect, and wherein the method further comprises determining an attribute of the directional sound effect.

6. The method according to any one of the preceding claims, further comprising, prior to acquiring (202) one or more conditions under which the data required for analyzing a first biological parameter of the subject is to be captured, the steps of:
acquiring initial data associated with at least one of the subject, a device used by the subject, and an application program used by the subject; and
determining whether the acquired initial data is sufficient for performing an analysis of the first biological parameter with at least one of a level of accuracy higher than a predetermined threshold and a level of speed higher than a predetermined threshold,
wherein acquiring (202) one or more conditions under which the data required for analyzing a first biological parameter of subject is to be captured is performed upon determining that the acquired initial data is not sufficient for performing an analysis of the first biological parameters with at least one of a level of accuracy higher than the predetermined threshold and a level of speed higher than the predetermined threshold.

7. The method according to claim 6, wherein the acquired initial data comprises at least one of: data associated with a preference of the subject, data associated with an environment of the subject, usage data of a device used by the subject, usage data of an application program used by the subject, a value of the second biological parameter, image data of a body part of the subject associated with the first biological parameter, and sound data generated by at least one of the subject, the environment of the subject, a device used by the subject, or an application program used by the subject.

8. The method according to any one of the preceding claims, wherein acquiring (206) at least one of: a current orientation of the body part of the subject, a current position of the body part of the subject, a current movement of a body part of the subject, and a current level of the second biological parameter of the subject comprises:
acquiring at least one of: an image of the body part of the subject and a sound of the environment of the subject;
detecting one or more physical features of the body part of the subject by performing analysis of the at least one of acquired image and a sound of the environment of the subject; and
determining, based on the detected one or more physical features, the at least one of: a current orientation of the body part of the subject, a current position of the body part of the subject, a current movement of a body part of the subject, and a current level of the second biological parameter of the subject.

9. The method according to any one of the preceding claims, further comprising:
acquiring feedback from the subject in response to the generated guidance, the feedback being associated with at least one of a movement of a body part of the subject and a third biological parameter of the subject;
determining whether an update of the guidance is required based on the acquired feedback; and
generating new guidance for the subject when it is determined that an update of the guidance is required.

10. The method according to any one of the preceding claims, wherein a biological parameter is associated with one of the physiological state of the subject and the psychological state of the subject.

11. The method according to any one of the preceding claims, wherein the data required for analyzing a first biological parameter of a subject comprises one or more images of the body part of the subject, and each of the one or more conditions under which data required for analyzing a first biological parameter of a subject is to be captured is associated with at least one of: a sharpness of the one or more images, a level of illumination of the body part of the subject, an angle at which the one or more images are captured, and an activity performed by the user during which the one or more images are captured.

12. A computer program product comprising a computer readable medium, the computer readable medium having computer readable code embodied therein, the computer readable code being configured such that, on execution by a suitable computer or processor, the computer or processor is caused to perform the method according to any one of claims 1 to 11.

13. A system (100) configured to provide guidance to a subject, the system comprising:
a user interface (110) configured to output one or more visual or audio elements;
a sensing unit (120) configured to capture data required for analyzing a first biological parameter of a subject; and
a control unit (130) configured to:
acquire one or more conditions under which the data required for analyzing the first biological parameter is to be captured;
determine, based on the one or more conditions, one or more requirements associated with at least one of: an orientation of a body part of the subject, a position of a body part of the subject, a movement of a body part of the subject, and a level of a second biological parameter of the subject;
acquire, based on the one or more requirements, at least one of: a current orientation of the body part of the subject, a current position of the body part of the subject, a current movement of a body part of the subject, and a current level of the second biological parameter of the subject;
determine a required adjustment of at least one of: an orientation of the body part of the subject, a position of the body part of the subject, and a second biological parameter of the subject, based on the respective requirement and the acquired information associated with the respective requirement;
generate guidance for the subject based on the determined required adjustment by performing at least one of:
generating a directional sound effect to be outputted by the user interface, wherein the directional sound effect is configured to induce the subject to achieve the required adjustment;
determining at least one of a relative position and an attribute of a visual element that is to be outputted by the user interface so as to induce the subject to achieve the required adjustment; and
adjusting a setting of the sensing unit to achieve the required adj ustment.

14. The system (100) according to claim 12, wherein the control unit (130) is further configured to, prior to acquiring one or more conditions under which the required data is to be captured, acquire initial data associated with at least one of the subject, a device used by the subject, and an application program used by the subject, and determine whether the acquired initial data is sufficient for performing an analysis of the first biological parameter with at least one of: a level of accuracy higher than a predetermined threshold and a level of speed higher than a predetermined threshold,
wherein the control unit is configured to acquire one or more conditions under which the data required for analyzing a first biological parameter of subject is to be captured when it is determined that the acquired initial data is not sufficient for performing an analysis of the first biological parameters with at least one of: a level of accuracy higher than the predetermined threshold and a level of speed higher than a predetermined threshold.

15. A device comprising a reflective component configured to reflect incident light, and the system (100) according to any one of claims 13 to 14.
